# EUROPÄISCHE PATENTANMELDUNG

(11) **EP 1 038 550 A2**
(43) Veröffentlichungstag der Anmeldung: **27.09.2000**
(21) Anmeldenummer: 00105693.6
(22) Anmeldetag: 17.03.2000
(51) Int. Cl.: A61N 5/06

(54) **Wärmekabine**

(30) Priorität: 22.03.1999 DE 19912609
(71) Anmelder: SEVRI - Saunabau Fritz Seebauer, 86633 Neuburg (DE)
(72) Erfinder: Gesierich, Roland, 86633 Neuburg (DE)
(74) Vertreter: Canzler, Rolf, Dipl.-Ing.

(57) **Zusammenfassung**

Die Erfindung betrifft eine Wärmekabine zum Bestrahlen des menschlichen Körpers mit Infrarotstrahlen, in welcher eine Sitz- oder Liegeeinrichtung sowie mindestens ein Infrarotstrahler angeordnet sind. Die Infrarotstrahler sind in einem definierten Abstand zu dem zu bestrahlenden Patienten angeordnet und in ihrer Strahlungsleistung auf diesen Abstand abgestimmt, wobei der Kopfbereich von der Bestrahlung ausgespart ist. Der Infrarotstrahler hat vorzugsweise eine Wellenlänge von 1,5 bis 3,5 µm.

## Beschreibung

Die Erfindung betrifft eine Wärmekabine zum Bestrahlen des menschlichen Körpers mit Infrarotstrahlen, in welcher eine Sitz- oder Liegeeinrichtung sowie mindestens ein Infrarotstrahler angeordnet sind.

Diese Wärmekabinen sind in Art der bekannten Saunakabinen aus Holz gebaut und bilden einen viereckigen Raum mit einer Bank als Sitzeinrichtung. Die Wand hinter der Sitzbank ist mit senkrechten Durchbrechungen versehen, in welchen ein oder mehrere Heizstrahler angeordnet sind. Um eine Berührung mit den Heizröhren zu verhindern, ist ein Schutzgitter zur Abdeckung der Durchbrechungen angebracht, welches von Holzleisten überbrückt wird, an die sich der Patient anlehnen kann. Ferner ist hinter den Heizstäben ein Edelstahlspiegel angebracht, meist in Dreieckform, um die Strahlung auf den Patienten zu richten und Strahlung nach rückwärts zu verhindern. Der Spiegel muß in einem gewissen Abstand vom Heizstab angeordnet werden, damit er sich nicht zu sehr erhitzt und zu Sekundärstrahlungen in Richtung Außenwand führt. Diese Strahler bauen deshalb in der Tiefe relativ groß, so daß die Dicke der Doppelwand der Kabine nicht ausreicht, um die Strahler mit dem Spiegel vollständig aufzunehmen. Sie ragen außen über die Wandfläche heraus.

Abgesehen davon, daß dies Platz benötigt, ist auch die durchbrochene Wand keine günstige Lösung. Bisweilen werden die aus der Wand herausragenden Spiegel nach außen verblendet. Dies ist jedoch aufwendig und führt dennoch zu Vorsprüngen und verhindert eine glatte Außenwand. Die gesamte Wand müßte daher auf einen Hohlraum von etwa 20 bis 30 cm verstärkt werden, um sowohl den Reflektor in einer glatten Außenwand aufnehmen zu können, als auch einen entsprechenden Abstand zum Schutzgitter einhalten zu können. Häufig werden deshalb derartige Strahler nicht nur in die Rückwand, sondern auch in die Ecken der Kabine eingebaut.

Durch die DE 298 17 647 U1 ist eine Behandlungskabine bekannt, die sich zur Aufgabe gestellt hat, eine Ganzkörperbestrahlung zu schaffen, was durch einen im wesentlichen für Infrarotstrahlen durchlässigen Sitz aus einem entsprechenden Material sowie auf eine auf dem Sitz sitzende Person gerichtete Infrarotbestrahlungseinrichtung erfolgen soll. Hierfür sind im Deckenbereich und im Fußbereich jeweils Infrarotlampen eingebaut.

Es hat sich gezeigt, daß diese bekannte Behandlungskabine nicht oder nur sehr unvollkommen die angestrebten Wirkungen erfüllt. Durch das vorgeschlagene Kunststoff-Material wird ein erheblicher Anteil der IR-Strahlung absorbiert, so daß nur ein kleiner Rest dieser Strahlung die zu bestrahlende Person erreicht. Da die Strahlung im Quadrat der Entfernung abnimmt, müssen ziemliche Leistungen installiert werden, um die zu bestrahlende Person überhaupt zu erreichen. Meist kommt es deshalb lediglich zu einer Wärmeentwicklung, so daß, wie in einer Saunakabine, nur eine indirekte Erwärmung und keine IR-Bestrahlung stattfindet. Diese Wärmeentwicklung an der Hautoberfläche darf allerdings nicht zu hoch werden, um Schädigungen der zu bestrahlenden Person zu vermeiden. Die verwendeten Heizstrahler sind außerdem wegen ihres Wellenlängenbereichs meist wenig für physiotherapeutische Zwecke geeignet, um ein Schwitzen von innen nach außen, wie bei einer körperlichen Anstrengung, zu erreichen.

Ein weiterer Nachteil dieser Heizstrahler ist außerdem, daß der zur Abschirmung der Wärme erforderliche Edelstahlspiegel nicht nur erheblichen Platzbedarf hat, sondern durch Absonderung von Tropfenkondensation beschlägt und verkrustet.

Der Erfindung liegt die Aufgabe zugrunde, eine Wärmekabine zu schaffen, die diese Nachteile vermeidet und bei der die physiotherapeutische Wirkung wesentlich verbessert ist.

Diese Aufgabe wird gemäß den Merkmalen der Ansprüche 1 und 15 gelöst.

Der Erfindung liegt die Erkenntnis zugrunde, daß nur ganz bestimmte Wellenlängen aus dem Infrarotbereich für die eingangs genannte physiotherapeutische Behandlung des menschlichen Körpers in Frage kommen. Außerdem kann diese Infrarotstrahlung auch nur dann richtig zur Wirkung kommen, wenn eine ganz bestimmte Entfernung zwischen dem Strahler und der zu bestrahlenden Person eingehalten und die Strahlungsleistung auf diesen Abstand abgestellt wird. Dadurch, daß die Infrarotstrahler in einem Abstand hinter der der zu bestrahlenden Person zugewandten Oberfläche der Sitz- oder Liegeeinrichtung angeordnet sind, wird automatisch ein definierter Abstand zum Patienten geschaffen, wodurch sich eine optimale Wirkung der Infrarotstrahlen ergibt. Da der Patient das Bedürfnis hat, sich an die Rückenlehne anzulehnen, bzw. mit der Liege- oder Sitzoberfläche zwangsläufig in Kontakt steht, ist auf denkbar einfachste Weise der für die IR-Bestrahlung erforderliche Abstand eingehalten. Durch die Durchbrechungen dieser Oberfläche kann die Infrarotstrahlung auf einfache Weise ungehindert den Patienten erreichen. Teure Materialien, die Infrarotstrahlen durchlassen, sowie der Absorptionsverlust bei diesen Materialien wird durch die Erfindung auf einfache Weise vermieden. Es hat sich gezeigt, daß ein Abstand zwischen 14 und 17 cm zwischen dem Strahler und der Oberfläche der Sitz- oder Liegeeinrichtung besonders günstig ist im Hinblick auf den Leistungsbedarf bei flächendeckender Wirkung. Der Abstand kann auch auf gewünschte Abstände einstellbar sein.

Durch die horizontale Anordnung der Infrarotstrahler kann die Lehne körpergerecht geformt und trotzdem der Abstand der Strahler exakt eingehalten werden. Weitere Einzelheiten der Erfindung werden anhand der Zeichnungen erläutert. Es zeigen:
- Figuren 1 und 2: in Seitensicht und in der Vordersicht die Anordnung einer Sitzeinrichtung mit Rückenlehne in einer Wärmekabine
- Figuren 3 und 4: eine Anordnung wie in den Figuren 1 und 2, jedoch mit körpergerecht geformter Sitzeinrichtung sowie horizontal angeordneten Infrarotstrahlern
- Figur 6: eine Wärmekabine in der Draufsicht

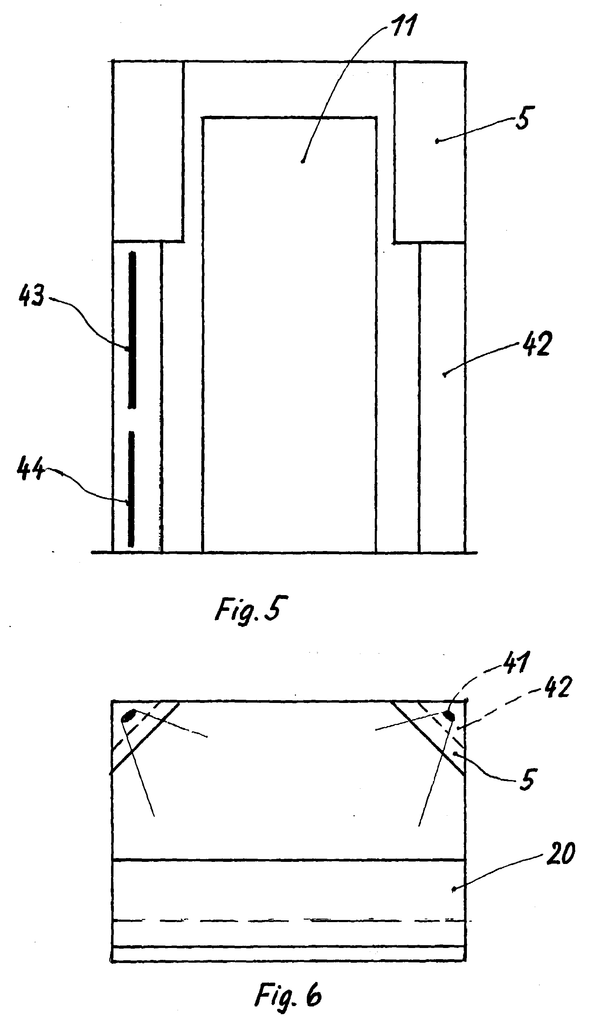

- Figur 5: eine Ansicht von Figur 6 von der Sitzbank aus im Aufriß

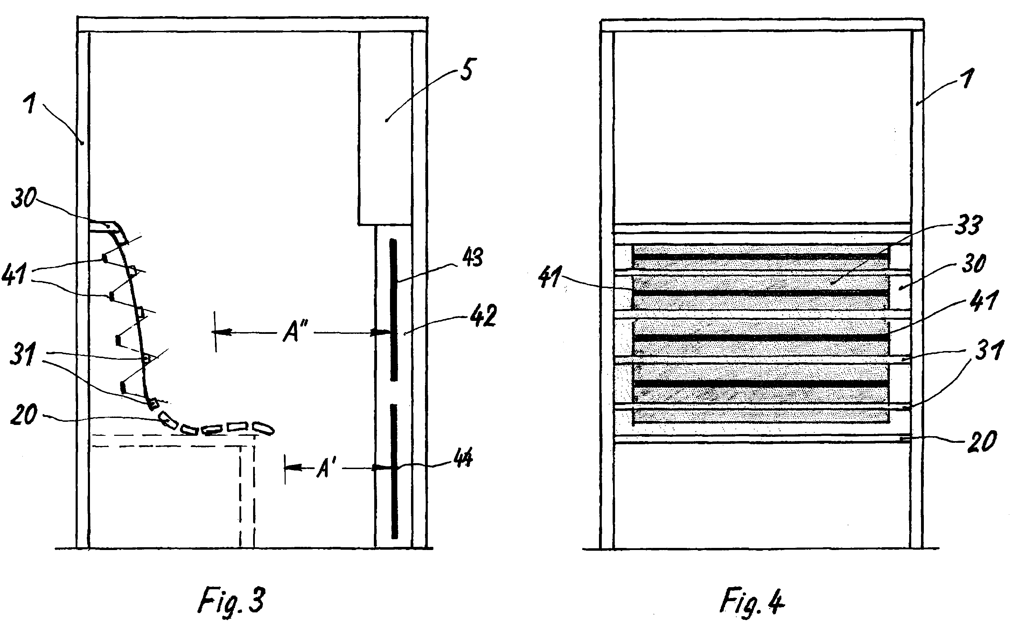

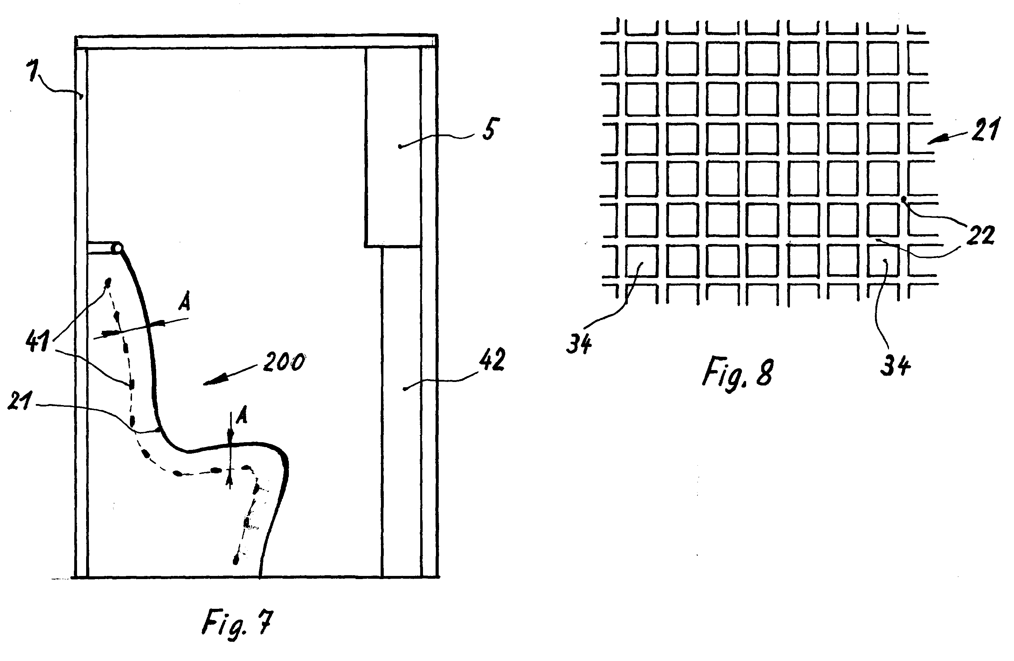

- Figuren 7 und 8: eine andere Ausführung der Sitzeinrichtung nach den Figuren 3 und 4.

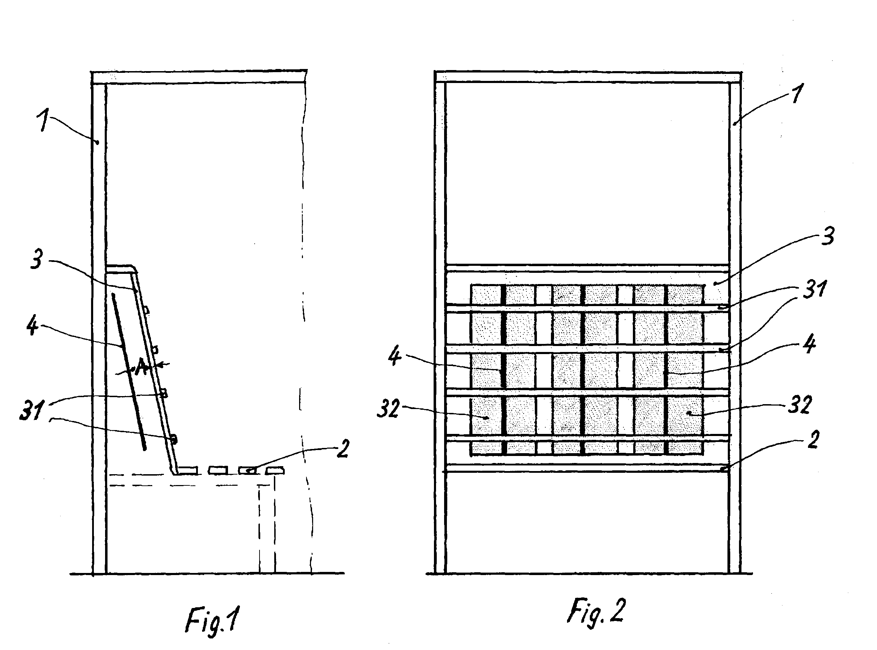

In Figur 1 ist in der Seitensicht eine Wärmekabine mit der Kabinenwand 1 und der Sitzeinrichtung 2 gezeigt. Die als Bank ausgebildete Sitzeinrichtung 2 weist eine Rückenlehne 3 auf, die senkrechte Durchbrechungen 32 aufweist, die durch Leisten 31 überquert sind. Hinter der Rückenlehne 3 sind in einem Abstand A zu der von der Rückenlehne 3 gebildeten Oberfläche Heizstäbe 4 angeordnet, die die Infrarotstrahlung erzeugen, die durch die Durchbrechungen 32 der Rückenlehne 3 austritt.

Damit die Strahlung nicht nach rückwärts erfolgen kann, ist wie üblich hinter jedem Heizstab 4 ein Reflektor (nicht abgebildet) angeordnet. Wie aus Figur 1 ersichtlich, ist der Heizstab 4 parallel zur Rückenlehne 3 in einem Abstand A angeordnet, so daß eine immer gleiche Strahlenintensität auf den Patienten wirkt, wenn dieser sich an die Rückenlehne 3 anlehnt.

Die Figuren 3 und 4 zeigen eine andere Ausführung der Erfindung, bei welcher die Sitzeinrichtung 20 zusammen mit der Rückenlehne 30 körpergerecht geformt ist. Die Rückenlehne 30 weist eine Durchbrechung 33 auf, die sich horizontal und vertikal fast über die gesamte Breite der Rückenlehne 30 erstreckt, jedoch von Leisten 31 überbrückt wird. Im Abstand A zu der von den Leisten 31 gebildeten Rückenlehnenoberfläche sind Heizstäbe 41 horizontal angeordnet. Zweckmäßig sind die Halterungen für die Heizstäbe 41 seitlich an dem Rahmen der Rückenlehne 30 befestigt, so daß trotz der gebogenen Form der Rückenlehne 30 für die Heizstäbe 41 der günstige Abstand A über die Oberfläche der Rückenlehne 30 eingehalten wird. Die Heizstäbe 41 sind somit parallel zu der gewölbten Oberfläche der Rückenlehne 30 angeordnet.

Der angedeutete Strahlungswinkel wird durch entsprechende Reflektoren hervorgerufen, die den Heizstäben 41 zugeordnet sind. Es hat sich als außerordentlich vorteilhaft erwiesen, sog. Zwillingsrohrstrahler zu verwenden, bei denen in zwei aneinanderliegenden Quarzglasröhren die Heizwendel zur Erzeugung der Infrarotstrahlung angeordnet sind. Die Anordnung eines Goldreflektors an der Rückseite des Strahlers erhöht dabei die wirksame Strahlung und verstärkt die Richtwirkung. Da der Reflektor praktisch in den Strahler integriert ist, ist dieser vor Verschmutzung oder Beschädigung geschützt. Diese äußerst platzsparende Bauweise ermöglicht es, die Lehnenkonstruktion schmal zu halten und dennoch keine Einschnitte in der Kabinenwand vorzunehmen. Diese Zwillingsrohrstrahler sind zwar als Heizstäbe für andere technische Zwecke schon bekannt (Heraeus-Infrarotstrahler - produktive Wärme als Programm). Es hat sich jedoch überraschenderweise gezeigt, daß diese Zwillingsrohrstrahler sich auf die Wellenlänge anpassen lassen, die für physiotherapeutische Zwecke besonders geeignet ist.

Aufgrund umfangreicher Untersuchungen wurde festgestellt, daß mit Wellenlängen von 0,76 µm bis 5 µm, insbesondere im Bereich von 1,5 bis 3,5 µm, sich eine hervorragende, therapeutische Strahlungswirkung auf den menschlichen Körper erzielen läßt. In diesem Wellenlängenbereich dringt die Infrarotstrahlung in die Hautschichten etwa 3 - 4 mm ein und erwärmt diese direkt, so daß erheblich geringere Umgebungstemperaturen, als beispielsweise in der Sauna, erforderlich sind, um den Patienten zum Schwitzen zu bringen. Dabei beruht die therapeutische Wirkung gerade darauf, daß die Hautoberfläche nicht durch aufgeheizte Luft erwärmt wird, sondern eine Erwärmung und Kreislaufanregung wie bei einer körperlichen Anstrengung erfolgt.

Desweiteren hat sich gezeigt, daß ein bestimmter Abstand A zum Körper der zu bestrahlenden Person eingehalten werden muß, um in diesem Strahlungsbereich die angestrebte therapeutische Wirkung lückenlos zu erzielen. Bedingt durch den Reflektor weisen die Strahler einen bestimmten Strahlungswinkel auf, so daß durch den Abstand A und den Strahlungswinkel die von einem Heizstab 4 oder 41 bestrahlte Fläche definiert wird. Entsprechend sind Heizstäbe 4 oder auch 41 so nebeneinander angeordnet, so daß eine Fläche gleicher Strahlungsintensität entsteht. Durch die entsprechende Gestaltung der Sitz- oder Liegeoberfläche entsteht zwangsläufig ein Kontakt mit dem Körper, wodurch der gewünschte Abstand A zu den Infrarotstrahlern in einfacher Weise durch den Patienten eingehalten wird. Dabei hat sich ein Abstand A zur Oberfläche der Sitz- oder Liegeeinrichtung von 14 bis 17 cm als optimal erwiesen, besonders auch im Hinblick auf die zu installierende Leistung.

Da die Infrarotstrahler nicht die gesamte Umgebungsluft aufheizen müssen, können sie mit einer wesentlich geringeren Leistungsaufnahme auskommen, um die gewünschte Strahlung in einem Wellenlängenbereich von 1,5 bis 3,5 µm zu erzeugen, und sind daher außerordentlich energiesparend. Die Energie wird zu über 80 % zur direkten Wärmebehandlung des Patienten benutzt und die Umgebungsluft nur sehr gering aufgeheizt. Durch den definierten Abstand A wirkt die Strahlung überall gleichmäßig intensiv.

Die horizontale Anordnung der Strahler 41 ermöglicht auch eine sich fast über die gesamte Breite und Höhe der Rückenlehne 30 erstreckende Durchbrechung 33, so daß der Patient nicht darauf achten muß, in einer bestimmten Lage vor der Durchbrechung 33 zu sitzen, wie dies bei den vertikal angeordneten Strahlern 4 in Figur 2 der Fall ist. Selbstverständlich könnte auch hier eine durchgehende Durchbrechung 33 sich über die Rückenlehne 30 erstrecken.

Die Durchbrechung muß nicht aus einer durchgehenden Öffnung bestehen, sondern kann sich auch aus vielen kleinen Durchbrechungen 34 zusammensetzen, wie beispielsweise in Figur 8 gezeigt. Die Oberfläche der Sitz- oder Liegeeinrichtung 200 wird somit aus einem Raster- oder Gitternetz 21 mit relativ schmalen Stegen 22 gebildet. Auf diese Weise wird eine Strahlendurchlässigkeit von über 80 % erreicht bei einer für den Patienten äußerst bequemen und komfortablen Sitz- oder Liegefläche. Zweckmäßig ist dieses Raster- oder Gitternetz 21 aus einem weichen Plastikmaterial hergestellt. Dieses Material läßt sich auch auf einfache Weise hygienisch sauberhalten.

In Figur 7 ist eine solche Sitzeinrichtung 200 dargestellt, die in ihrer gesamten Oberfläche ein durchgehendes Gitternetz 21 mit Durchbrechungen 34 aufweist. Dadurch ist nicht nur der Rückenbereich, sondern auch der Sitz- und Beinbereich einer Bestrahlung zugänglich. Auch hier wird gemäß der Erfindung ein gleichbleibender Abstand A zu den Heizstäben 41 stets eingehalten, auch wenn die Sitzeinrichtung der Körperform angepaßt ist. Gerade durch diese Anpassung der Sitz- oder Liegeeinrichtung hat der Patient zwangsläufig und ohne daß er besonders darauf achten muß, stets den richtigen Abstand zur Strahlungseinrichtung 41.

Um auch eine Bestrahlung von vorn zu bewirken, sind in den Ecken der Kabine weitere Strahler 42 in bekannter Weise angeordnet. Diese Eckstrahler 42 werden nur bis zu einer Höhe der Rückenlehnenoberkante etwa benötigt, da die Bestrahlung des Kopfes unerwünscht ist. Durch diese Eckstrahler 42 läßt sich der optimale Abstand von 14 bis 17 cm zwar nicht generell einhalten, jedoch können diese Strahler mit einer höheren Leistung ausgestattet werden, so daß die abgegebene Strahlung den Patienten mit der gewünschten Intensität erreicht. Da im Beinbereich der Abstand A' wesentlich geringer ist als im Bereich des Oberkörpers, weisen diese Eckstrahler 42 unterteilt Heizelemente 43 und 44 auf, die jeweils auf die erforderliche Distanz A' und A'' in ihrer Leistung und Strahlungsfähigkeit ausgelegt sind. Diese Eckstrahler 42 sind in einem Gehäuse vertikal so angeordnet, so daß sie gleichzeitig auch Teil eines Belüftungssystemes für die Kabine sind durch ihre kaminartige Wirkung: Die erzeugte Wärme steigt wie in einem Kamin nach oben und wird durch eine (nicht gezeigte) Lüftungsöffnung im Dach der Kabine abgeführt, während Frischluft im Bodenbereich nachströmt.

Wie in Figur 5 dargestellt, sind über diesen Eckstrahlern 42 Leuchtflächen 5 vorgesehen, um gleichzeitig mit der Infrarotbestrahlung eine Lichtbehandlung durchzuführen. Da die Leuchtdichte für derartige Biolichtbehandlungen wenigstens 2.000 lux betragen muß, wird dadurch die Größe und damit der Platzbedarf der Lichtfläche 5 bestimmt. Die Platzverhältnisse neben der Türöffnung 11 eignen sich für die Anordnung derartiger Lichtflächen 5 über Eck besonders gut.

Die Erfindung ist anhand einer Sitzeinrichtung mit Rückenlehne beschrieben. Selbstverständlich ist es auch möglich, die Erfindung bei Liegeeinrichtungen zu verwenden. Auch hier wird der Patient durch die Liegenoberfläche fixiert, und es lassen sich Heizstäbe 41 in einem definierten Abstand A parallel zu dieser Liegenoberfläche anordnen. Ist die Liege der Körperform angepaßt, so bietet sich auch hier die horizontale Anordnung der Heizstäbe 41 an, d.h. bei der Liege bedeutet dies eine Anordnung quer zur Längsachse der Liege. Zweckmäßig sind die Heizstäbe 41 mit ihrer Halterung am Rahmen der Sitz- oder Liegeeinrichtung befestigt. Diese Anordnung hat besonders den Vorteil, daß auch bei Verstellung der Liege die Heizstäbe 41 mitverstellt werden, so daß stets der optimale Abstand A zum Patienten eingehalten wird. Durch Hochklappen der Liege kann diese zur Sitzeinrichtung werden, und umgekehrt kann die Sitzeinrichtung durch Herunterklappen der Rückenlehne zur Liege werden.

### Bezugszeichenliste

- 1: Kabinenwand
- 2,20,200: Sitzeinrichtung
- 21: Gitteroberfläche
- 3,30: Rückenlehne
- 31: Leisten
- 32,33,34: Durchbrechung
- 4,41,43,44: Heizstab
- 42: Eckstrahler
- 5: Lichtfläche
- 11: Türöffnung
- A: Abstand

## Patentansprüche

1. Wärmekabine zum Bestrahlen des menschlichen Körpers mit Infrarotstrahlen, in welcher eine Sitz- oder Liegeeinrichtung mit einer dem Körper des Patienten zugewandten Sitz- oder Liegeoberfläche sowie mindestens ein Infrarotstrahler angeordnet sind, **dadurch gekennzeichnet, daß** die Infrarotstrahler (4;41;43;44) in einem definierten Abstand (A;A';A'') zu dem zu bestrahlenden Patienten angeordnet und in ihrer Strahlungsleistung auf diesen Abstand (A;A';A'') jeweils so abgestimmt sind, daß bei einem gegebenen Strahlungswinkel eine gewünschte Strahlungsintensität gleichmäßig über die Körperoberfläche des Patienten erreicht wird, wobei der Kopfbereich von der Bestrahlung ausgespart ist.

2. Vorrichtung nach Anspruch 1, **dadurch gekennzeichnet, daß** die dem Körper des Patienten zugewandte Oberfläche der Sitz- oder Liegeeinrichtung (2,20;200) wenigstens eine Durchbrechung (32;33;34) aufweist, und hinter dieser Oberfläche im Bereich der Durchbrechung (32; 33; 34) in einem Abstand (A) zu dieser Oberfläche die Infrarotstrahler (4; 41) parallel zu der durch die Liege- oder Sitzeinrichtung (2;20;200) gebildeten Oberfläche angeordnet sind.

3. Vorrichtung nach einem der Ansprüche 1 oder 2, **dadurch gekennzeichnet, daß** die Durchbrechung (33) sich durchgehend über die Breite und Höhe der Rückenlehne (30) erstreckt.

4. Vorrichtung nach einen oder mehreren der vorstehenden Ansprüche, **dadurch gekennzeichnet, daß** die Oberfläche (21) der Sitz- oder Liegeeinrichtung eine Vielzahl von Durchbrechungen in Art einer Raster- oder Netzstruktur aufweist.

5. Vorrichtung nach einem oder mehreren der vorstehenden Ansprüche, **dadurch gekennzeichnet, daß** der Abstand (A) der IR-Strahler (4; 41) von der Oberfläche der Siotz- oder Liegeeinrichtung (2;20;200) etwa 14 bis 17 cm beträgt.

6. Vorrichtung nach einem oder mehreren der vorstehenden Ansprüche, **dadurch gekennzeichnet, daß** der Abstand (A;A';A'')) einstellbar ist.

7. Vorrichtung nach einem oder mehreren der vorstehenden Ansprüche, **dadurch gekennzeichnet, daß** die IR-Strahler (41) horizontal angeordnet sind.

8. Vorrichtung nach einem oder mehreren der vorstehenden Ansprüche, **dadurch gekennzeichnet, daß** die Infrarotstrahler (41) quer zur Krümmungsrichtung der Oberfläche der Sitz- oder Liegeeinrichtung (20;200) angeordnet sind.

9. Vorrichtung nach einem oder mehreren der vorstehenden Ansprüche, **dadurch gekennzeichnet, daß** gegenüber der Sitz- oder Liegeeinrichtung (2;20;200) Infrarotstrahler (43;44) angeordnet sind, die in ihrer Strahlungsleistung auf den jeweiligen Abstand (A';A'') zu dem zu bestrahlenden Patienten abgestimmt sind.

10. Vorrichtung nach einem oder mehreren der vorstehenden Ansprüche, **dadurch gekennzeichnet, daß** Eckstrahler (42) vorgesehen sind, die mit Heizstäben (43;44) ausgestattet sind, die entsprechend ihrem Abstand (A';A'') zu dem zu bestrahlenden Patienten unterschiedliche Strahlungsleistungen aufweisen.

11. Vorrichtung nach einem oder mehreren der vorstehenden Ansprüche, **dadurch gekennzeichnet, daß** die Infrarotstrahler (41;43;44) so angeordnet und ausgebildet sind, daß nur die Körperteile erreicht werden, deren Bestrahlung gewünscht wird.

12. Vorrichtung nach einem oder mehreren der vorstehenden Ansprüche, **dadurch gekennzeichnet, daß** der Infrarotstrahler (4;41;43;44) einen integrierten Reflektor besitzt.

13. Vorrichtung nach einem oder mehreren der vorstehenden Ansprüche, **dadurch gekennzeichnet, daß** der Infrarotstrahler (4;41;43;44) eine Quarzglasröhre aufweist, die den Heizdraht umgibt und in welcher hinter dem Heizdraht ein Reflektor angeordnet ist.

14. Vorrichtung nach einem oder mehreren der vorstehenden Ansprüche, **dadurch gekennzeichnet, daß** der Infrarotstrahler (4;41;43;44) als Zwillingsrohrstrahler ausgebildet ist.

15. Wärmekabine zum Bestrahlen des menschlichen Körpers mit Infrarotstrahlen, in welcher eine Sitz- oder Liegeeinrichtung mit einer dem Körper des Patienten zugewandten Sitz- oder Liegeoberfläche sowie mindestens ein Infrarotstrahler angeordnet sind, insbesondere nach einem oder mehreren der vorstehenden Ansprüche, **dadurch gekennzeichnet, daß** der Infrarotstrahler (4;41;43;44) eine Wellenlänge von 0.76 bis 5 µm, vorzugsweise von 1,5 bis 3,5 µm besitzt.

16. Vorrichtung nach einem oder mehreren der Ansprüche 1 bis 6, **dadurch gekennzeichnet, daß** gegenüber der Sitzeinrichtung in Lehnenhöhe beginnend bis zur Decke Biolichtflächen (5) mit einer Leuchtdichte von wenigstens 2.000 lux angeordnet sind.
